# EUROPEAN PATENT APPLICATION

(11) **EP 3 097 803 A1**
(43) Date of publication of application: **30.11.2016**
(21) Application number: 15169225.8
(22) Date of filing: 26.05.2015
(51) Int. Cl.: A24F 47/00, A61M 15/06

(54) **ELECTRONIC SMOKING DEVICE**

(71) Applicant: Fontem Holdings 1 B.V., 1083 HN Amsterdam (NL)
(72) Inventor: Borkovec, Vaclav, 22761 Hamburg (DE); Tschierske, Nicole, 22761 Hamburg (DE); Biel, Stefan, 22761 Hamburg (DE)
(74) Representative: Gulde & Partner

(57) **Abstract**

An atomizer/liquid reservoir portion (14) is described for forming a cylindrical tube together with a battery portion (12) of an electronic smoking device. The atomizer/liquid reservoir portion (14) has an air passage and an air inhalation port (36) provided at the end of the atomizer/liquid reservoir portion (14) remote from the other end for attachment to the battery portion (12), wherein the atomizer/liquid reservoir portion (14) further has a receiving element (40) for receiving a powder for release into the air passage (32) between the atomizer (26) and the air inhalation port (36).

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), usually has a housing accommodating an electric power source (e.g. a single use battery or a rechargeable battery), and an electrically operable atomizer. The atomizer vaporizes or atomizes liquid supplied from a reservoir and provides vaporized or atomized liquid as an aerosol. Control electronics control the activation of the atomizer. In many electronic cigarettes, an airflow sensor is provided within the electronic smoking device which detects a user puffing on the device (e.g., by sensing an under-pressure or an air flow pattern through the device). The airflow sensor indicates or signals the puff to the control electronics. Alternatively, a button may be used to switch on the electronic smoking device to generate a puff. When a puff is detected, the control electronics supplies electrical power to the atomizer thereby creating vaporized liquid as an aerosol.

For flavoring, food grade aromas are sometimes mixed into the e-Liquid where they are vaporized during the atomization process, followed by a condensation stage that produces an aerosol which is inhaled. The aerosol particles are deposited along the respiratory tract according to their size.

### SUMMARY OF THE INVENTION

In one aspect, an electronic smoking device is provided which comprises a liquid reservoir and a powder reservoir where the powder reservoir contains a powder comprising at least one olfactory, gustatory, stimulating and/or nutritive active ingredient. In another aspect there is provided a cartomizer for an electronic smoking device comprising an atomizer, a liquid reservoir and a powder reservoir containing a powder comprising at least one olfactory, gustatory, stimulating and/or nutritive active ingredient. In a further aspect there is provided a liquid reservoir for use with an electronic smoking device wherein the liquid reservoir additionally comprises a powder reservoir containing a powder comprising at least one olfactory, gustatory, stimulating and/or nutritive active ingredient.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
Figure 1 is a schematic cross-sectional illustration of an exemplary electronic smoking device according to an embodiment;
Figure 2 is a schematic cross-sectional illustration of an exemplary electronic smoking device according to another embodiment;
Figure 3 is a schematic cross-sectional illustration of an exemplary cartomizer according to an embodiment;
Figure 4 is a schematic cross-sectional illustration of an exemplary cartomizer according to another embodiment; and
Figure 5 is a schematic cross-sectional illustration of an exemplary cartomizer according to yet another embodiment;
Figure 6 is a schematic cross-sectional illustration of an exemplary cartomizer according to even yet another embodiment; and
Figure 7 is a schematic cross-sectional illustration of an exemplary cartridge according to an embodiment; and
Figure 8 is a schematic cross-sectional illustration of an exemplary cartridge according to another embodiment.

### DESCRIPTION OF THE PREFERRED EMBODIMENTS

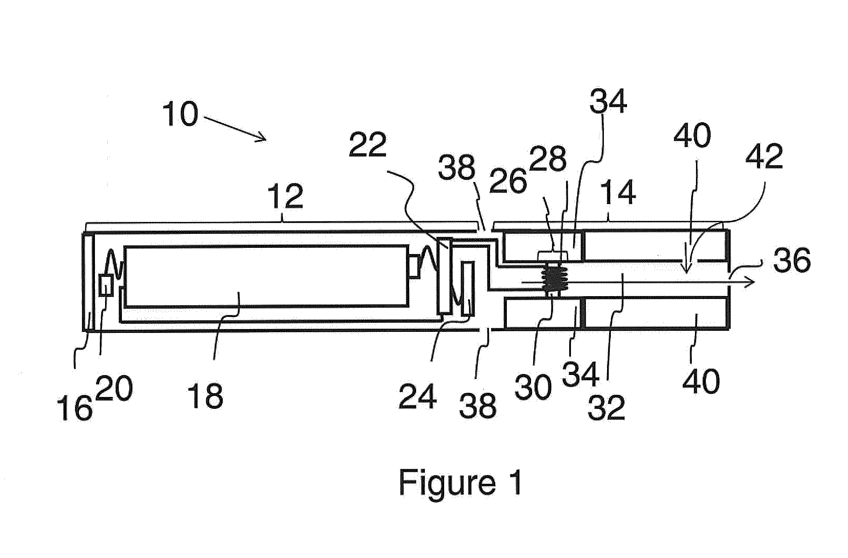

Throughout the following, an electronic smoking device according to an embodiment of the invention will be exemplarily described with reference to an e-cigarette. As is shown in Figure 1, an e-cigarette 10 typically has a housing comprising a cylindrical hollow tube having an end cap 16. The cylindrical hollow tube may be single piece or a multiple piece tube. In Figure 1, the cylindrical hollow tube is shown as a two piece structure having a battery portion 12 and an atomizer/liquid reservoir portion 14. Together the battery portion 12 and the atomizer/liquid reservoir portion 14 form a cylindrical tube which is approximately the same size and shape as a conventional cigarette, typically about 100 mm with a 7.5 mm diameter, although lengths may range from 70 to 150 or 180 mm, and diameters from 5 to 30 mm.

The battery portion 12 and atomizer/liquid reservoir portion 14 are typically made of steel or hardwearing plastic and act together with the end caps to provide a housing to contain the components of the e-cigarette 10. The battery portion 12 and an atomizer/liquid reservoir portion 14 may be configured to fit together by a friction push fit, a snap fit, or a bayonet attachment, magnetic fit, or screw threads. The end cap 16 is provided at the front end of the battery portion 12. The end cap 16 may be made from translucent plastic or other translucent material to allow an LED 20 positioned near the end cap to emit light through the end cap. The end cap can be made of metal or other materials that do not allow light to pass.

An air inlet may be provided in the end cap, at the edge of the inlet next to the cylindrical hollow tube, anywhere along the length of the cylindrical hollow tube, or at the connection of the battery portion 12 and the atomizer/liquid reservoir portion 14. Figure 1 shows a pair of air inlets 38 provided at the intersection between the battery portion 12 and the atomizer/liquid reservoir portion 14.

A battery 18, a light emitting diode (LED) 20, control electronics 22 and optionally an airflow sensor 24 are provided within the cylindrical hollow tube battery portion 12. The battery 18 is electrically connected to the control electronics 22, which are electrically connected to the LED 20 and the airflow sensor 24. In this example the LED 20 is at the front end of the battery portion 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure such a microphone switch including a deformable membrane which is caused to move by variations in air pressure. Alternatively the sensor may be a Hall element or an electro-mechanical sensor.

The control electronics 22 are also connected to an atomizer 26. In the example shown, the atomizer 26 includes a heating coil 28 which is wrapped around a wick 30 extending across an air passage of the atomizer/liquid reservoir portion 14. The air passage is a central passage 32, but this is optional. The coil 28 may be positioned anywhere in the atomizer 26 and may be transverse or parallel to the liquid reservoir 34. The wick 30 and heating coil 28 do not completely block the central passage 32. Rather an air gap is provided on either side of the heating coil 28 enabling air to flow past the heating coil 28 and the wick 30. The atomizer may alternatively use other forms of heating elements, such as ceramic heaters, or fiber or mesh material heaters. Nonresistance heating elements such as sonic, piezo and jet spray may also be used in the atomizer in place of the heating coil.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other embodiments the liquid reservoir 34 may comprise a toroidal cavity arranged to be filled with liquid and with the ends of the wick 30 extending into the toroidal cavity.

An air inhalation port 36 is provided at the back end of the atomizer/liquid reservoir portion 14 remote from the end cap 16. The inhalation port 36 may be formed from the cylindrical hollow tube atomizer/liquid reservoir portion 14 or maybe formed in an end cap.

In use, a user sucks on the e-cigarette 10. This causes air to be drawn into the e-cigarette 10 via one or more air inlets, such as air inlets 38 and to be drawn through the central passage 32, as indicated by the horizontal arrow, towards the air inhalation port 36. The change in air pressure which arises is detected by the airflow sensor 24 which generates an electrical signal that is passed to the control electronics 22. In response to the signal, the control electronics 22 activate the heating coil 28 which causes liquid present in the wick 30 to be vaporized creating an aerosol (which may comprise gaseous and liquid components) within the central passage 32. As the user continues to suck on the e-cigarette 10, this aerosol is drawn through the central passage 32 and inhaled by the user. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid reservoir 34 by capillary action and thus is available to be converted into an aerosol through subsequent activation of the heating coil 28.

Some e-cigarettes are intended to be disposable and the electric power in the battery 18 is intended to be sufficient to vaporize the liquid contained within the liquid reservoir 34 after which the e-cigarette 10 is thrown away. In other embodiments the battery 18 is rechargeable and the liquid reservoir is refillable. In the cases where the liquid reservoir 34 is a toroidal cavity, this may be achieved by refilling the liquid reservoir via a refill port. In other embodiments the atomizer/liquid reservoir portion 14 of the e-cigarette 10 is detachable from the battery portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit at least comprising the atomizer 26 and the liquid reservoir 34 is called a cartomizer.

The new liquid reservoir 34 may be in the form of a cartomizer having a central passage 32 through which a user inhales aerosol. In other embodiments, aerosol may flow around the exterior of the cartomizer to an air inhalation port 36.

Of course, in addition to the above description of the structure and function of a typical e-cigarette 10, variations also exist. For example, the LED 20 may be omitted. The airflow sensor 24 may be placed adjacent the end cap 16 rather than in the middle of the e-cigarette. The airflow sensor 24 may be replaced with a switch which enables a user to activate the e-cigarette manually rather than in response to the detection of a change in air flow or air pressure.

Different types of atomizers may be used. Thus for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively the atomizer may use a piezoelectric atomizer to create an aerosol either in combination or in the absence of a heater.

In the exemplary embodiment, the central passage 32 is further surrounded by a powder reservoir 40 with one or more openings 42 towards the central passage. The openings are arranged between the atomizer 26 and the inhalation port 36. Within the powder store 40 a powder can be stored. For instance, a powder of solid particles comprising at least one olfactory, gustatory, stimulating (e.g. the central nervous system) and/or nutritive active ingredient (e.g. one or more of: flavor(s), tastant(s), caffeine, carbohydrate(s), vitamin(s) and mineral(s)) coated and/or mixed with an effervescent material can be stored. Such powder is also known as dry powder. When the effervescent material comes in contact with a humidity level common to oral cavity, e.g. a humidity level common to the mouth, the effervescent material starts to effervesce to form gas bubbles containing the at least one olfactory, gustatory, stimulating (e.g. the central nervous system) and/or nutritive active ingredient and dispersing within the oral cavity by means of controlling the size of these particles. Larger particles will impact the back of the throat when drawn into the mouth and stick to the mucous lining of the throat, rather then being inhaled into the deep lung which is not desirable.

Additionally, these particles will be delivered mixed or coated with an effervescent material that reacts when absorbed into the mucous lining of the mouth and throat, causing a reaction that produces gas bubbles which provide a pleasant sensory experience that is completely new to the industry. Furthermore, by depositing the at least one olfactory, gustatory, stimulating (e.g. the central nervous system) and/or nutritive active ingredient in the correct part of the respiratory tract to maximize, e.g., flavor and taste perception, the flavor release will be higher than with current electronic cigarettes, or the same but requiring less material for the same effect.

Figure 1 shows a single opening only but more than one opening is possible. Two or more openings may be present.

Thus, when the user sucks on the atomizer/liquid reservoir portion 14 of the e-cigarette 10 not only an aerosol is generated which streams through the central passage 32, as indicated by the horizontal arrow, towards the inhalation port 36 but further some powder is entrained into the aerosol flow through the opening 42, as indicated by the vertical arrow, due to a pressure difference between the powder reservoir 40 and the central passage 32 caused by the user sucking. The resulting flow of air loaded with droplets and solid particles then is inhaled by the user wherein the solid active ingredient particles are dispersed in the mouth and/or throat while the nicotine from the droplets of the aerosol are delivered to the lungs where they are provided for resorption. This provides the user with, for instance, an increased olfactory and/or gustatory impression, without reducing the transfer of nicotine to the blood via the lung.

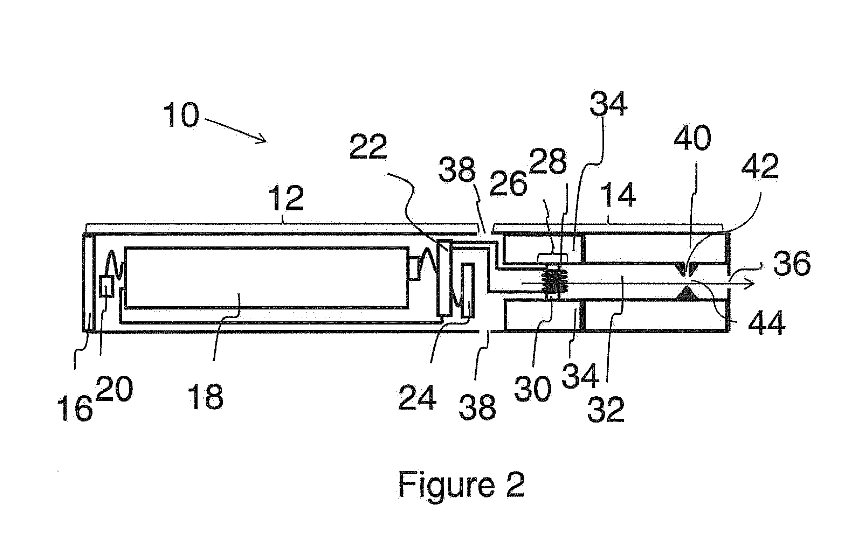

Figure 2 shows an exemplary embodiment where the central passage 32 has a narrowing or bottleneck 44 between the atomizer 26 and the inhalation port 36. The opening 42 is arranged such that the powder is released into the narrowing 44. However, the narrowing is optional.

Thus, when the user sucks on the atomizer/liquid reservoir portion 14 of the e-cigarette 10 not only an aerosol is generated which streams through the central passage 32, as indicated by the horizontal arrow, towards the inhalation port 36 but further some powder is entrained into the aerosol flow through the opening 42 due to the Bernoulli effect arising due to the presence of the narrowing 40, i.e. a pressure difference between the powder reservoir 40 and the central passage 32 caused by the user sucking as well as the narrowing 40. The resulting flow of air loaded with droplets and solid particles then is inhaled by the user wherein the solid particles are dispersed in the mouth and/or throat as described while the nicotine from the droplets of the aerosol is provided for resorption in the lung. This provides the user with a peak of blood plasma nicotine similar to conventional cigarettes, and additionally the novel sensory effect of a olfactory, gustatory, stimulating and/or nutritive effervescence in the mouth and optionally the throat that mimics a higher then delivered dose of nicotine.

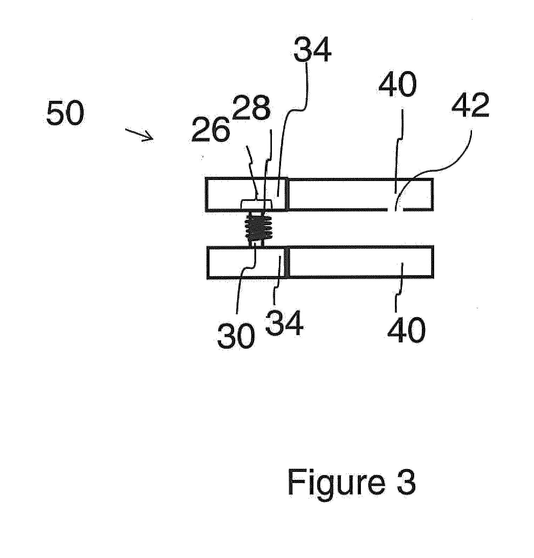

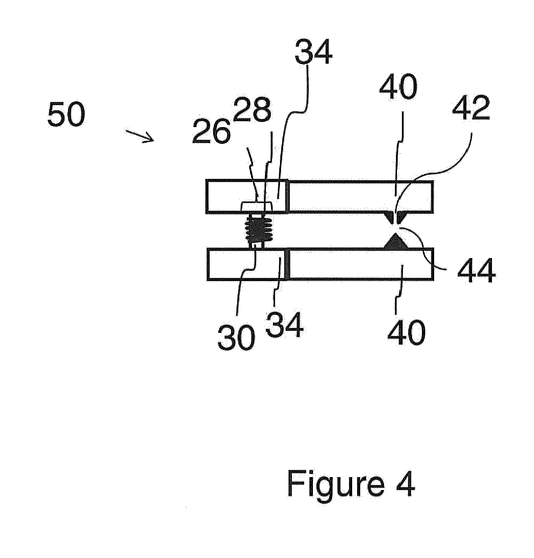

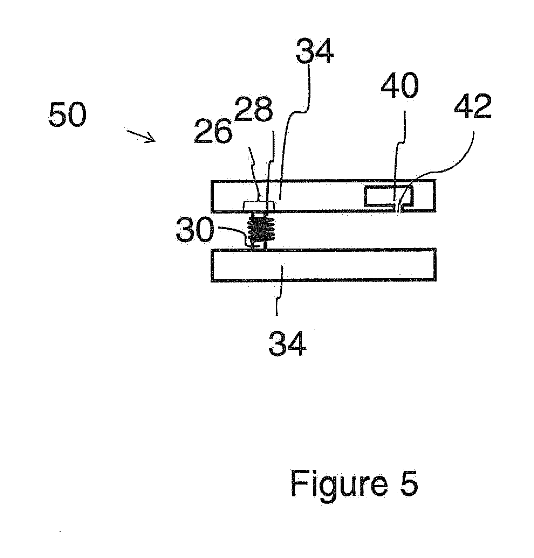

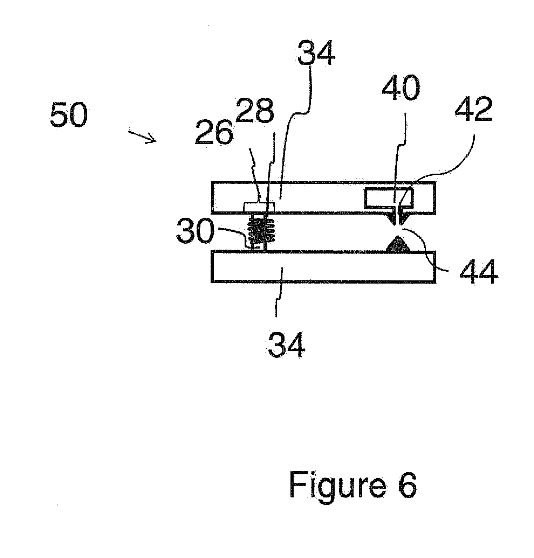

In some embodiments the powder reservoir 40 is part of a cartomizer 50 further comprising a liquid reservoir 34. Examples of these embodiments are depicted schematically in Figures 3, 4, 5 and 6. Figures 3 and 5 show exemplary embodiments of cartomizer 50 where no narrowing in the central passage 32 is present, and Figures 4 and 6 show exemplary embodiments of cartomizer 50 with a narrowing 40 in the central passage 32.

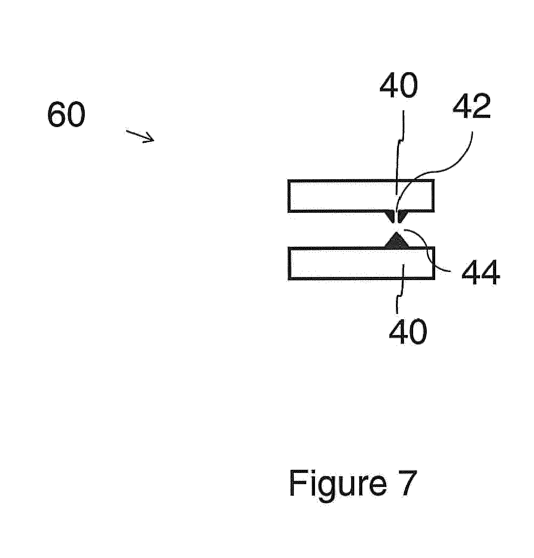

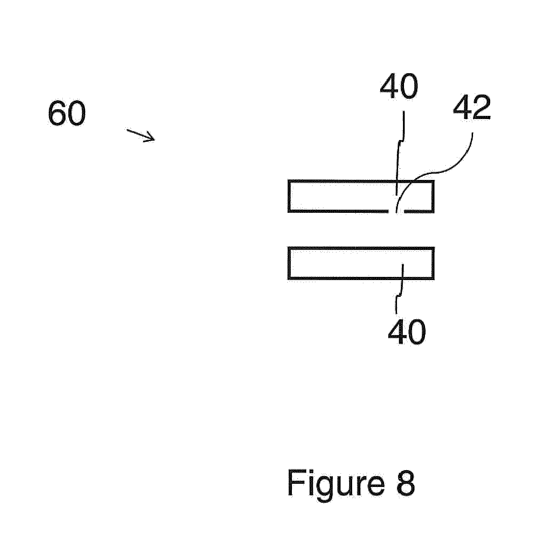

As shown schematically in Figs. 7 and 8, in some embodiments the powder reservoir 40 form part of a cartridge 60. Figure 7 shows an exemplary embodiment of the cartridge 60 where no narrowing in the central passage 32 is present and Figure 8 shows another exemplary embodiment of the cartridge 60 with a narrowing 40 in the central passage.

In case of a cartomizer 50 comprising the two reservoirs 34, 40, there are embodiments where the amount of powder in the powder reservoir 40 is provided in a ratio to the amount of liquid in the liquid reservoir 34 but this again is optional. Among the embodiments having reservoirs 34, 40 where the size of the reservoirs 34, 40 is such that the amount of air flow required for emptying the liquid reservoir 34 empties or substantially empties the powder reservoir 40, too. In some embodiments the powder reservoir 40 maybe such that when the liquid reservoir is emptied some powder (e.g., up to 10% of the original content of the powder reservoir 40) remains. This embodiment is designed so that the liquid reservoir 34 is emptied prior to the powder reservoir 40, so that in use a user will therefore normally be ensured of obtaining a mix of atomized liquid and powder when inhaling from the e-cigarette 10.

In some embodiments the e-cigarette 10 is intended to be disposable and the electric power in the battery 18 is intended to be sufficient to vaporize the liquid contained within the liquid reservoir 34 so the e-cigarette 10 is thrown away once both, the liquid reservoir and the powder reservoir, are empty. In other embodiments the battery 18 is rechargeable and an element or elements is provided to replenish the liquid supply as well as the powder reservoir. In the cases where the liquid reservoir 34 and the powder reservoir 40 are toroidal cavities, this may be achieved by providing refill ports and refilling the cavities with liquid and powder via the refill ports. In other embodiments the atomizer/liquid reservoir portion 14 of the e-cigarette 10 is detachable from the battery portion 12 and a new atomizer/liquid reservoir portion 14 can be fitted with a new liquid reservoir 34 and a new powder reservoir 40 thereby replenishing the supply of liquid and powder.

The new powder reservoir 40 may be in the form of cartridge detachable from the liquid reservoir 34 enabling the powder reservoir 40 to be replenished independently from the liquid reservoir 34. The liquid reservoir 34 may comprise a refillable liquid reservoir 34. Thus in use a user may fill the liquid reservoir 34 and insert a cartridge containing a powder reservoir 40 in to the electronic cigarette 10. The user may then use the electronic cigarette 10 until the liquid in the liquid reservoir 34 is depleted after which the liquid reservoir 34 may be refilled and a new powder reservoir 40 may be inserted.

The cartridge may be provided with a central passage 32 through which a user inhales aerosol loaded with powder generated by the e-cigarette 10, with an opening 42 of the cartridge towards the central passage 32. In other embodiments, rather than inhaling aerosol powder mix via a central passage 32, the cartridge may block the central portion of the e-cigarette 10 and generated aerosol powder mix may be directed in channels extending around the exterior of the cartridge to an air inhalation port 36 for inhalation. In this embodiment, the powder reservoir 40 may comprise one or more openings into such an exterior channel.

In summary, in some embodiments a cartomizer 50 for an atomizer/liquid reservoir portion 14 of an electronic smoking device comprises a liquid reservoir 34 for storing a liquid and an atomizer 26 for releasing an aerosol of the liquid from the liquid reservoir 34 into an air passage. The cartomizer 50 further comprises a powder reservoir 40 containing a powder comprising at least one olfactory, gustatory, stimulating and/or nutritive active ingredient (e.g.one or more of: flavor(s), tastant(s), caffeine, carbohydrate(s), vitamin(s) and mineral(s)). Optionally, the powder may comprise solid particles of flavor(s) and/or tastant(s) mixed with and/or coated with an effervescent material. The powder reservoir 40 may have one or more openings 42. Such openings 42 may be sealed with a removable seal.

Alternatively, a cartridge 60 may be provided for an atomizer/liquid reservoir portion 14 of an electronic smoking device, wherein the cartridge 60 comprises a powder reservoir 40 containing a powder comprising at least one olfactory, gustatory, stimulating and/or nutritive active ingredient (e.g.one or more flavor(s), tastant(s), caffeine, carbohydrate(s), vitamin(s) and mineral(s)).

Yet other embodiments concern electronic smoking devices 10 which include an atomizer/liquid reservoir portion 14 and a battery portion 12 of an electronic smoking device which together forms a cylindrical tube. In such embodiments the atomizer/liquid reservoir portion 14 may have an air passage. An air inhalation port 36 may be provided at the end of the atomizer/liquid reservoir portion 14 remote from the other end for attachment to the battery portion 12. The atomizer/liquid reservoir portion 14 may further include a receiving portion for receiving a powder for release into the air passage 32 between the atomizer 26 and the air inhalation port 36.

The receiving portion may be a powder reservoir 40 filled with a powder comprising at least one olfactory, gustatory, stimulating and/or nutritive active ingredient (e.g.one or more flavor(s), tastant(s), caffeine, carbohydrate(s), vitamin(s) and mineral(s)). The powder reservoir 40 may have at least one opening 42 to the air passage 32.

The air passage 32 may have a narrowing 44 between the atomizer 26 and the air inhalation port 36, the opening 42 being arranged such that the powder is released into the narrowing 44.

The powder may comprise solid particles of at least one olfactory, gustatory, stimulating and/or nutritive active ingredient (e.g.one or more of: flavor(s), tastant(s), caffeine, carbohydrate(s), vitamin(s) and mineral(s)) mixed and/or coated with an effervescent material responsive to humidity as commonly found in the mouth and/or throat of humans or other mammals. The powder may be released in response to a user sucking at the air inhalation port 36. The powder may be released by a pressure difference.

The atomizer/liquid reservoir portion 14 may further comprise a liquid reservoir 34 for storing a liquid and an atomizer 26 for releasing an aerosol of the liquid from the liquid reservoir 34 into the air passage wherein the receiving element 40 is configured for receiving a cartridge 60 comprising a powder reservoir 40 filled with a powder comprising at least one olfactory, gustatory, stimulating and/or nutritive active ingredient (e.g.one or more flavor(s), tastant(s), caffeine, carbohydrate(s), vitamin(s) and mineral(s)).

Or, the receiving element 40 may be configured for receiving a cartomizer 50 comprising a liquid reservoir 34 for storing a liquid and an atomizer 26 for releasing an aerosol of the liquid from the liquid reservoir 34 into an air passage wherein the cartomizer further comprises a powder reservoir 40 filled with a powder comprising at least one olfactory, gustatory, stimulating and/or nutritive active ingredient (e.g.one or more of: flavor(s), tastant(s), caffeine, carbohydrate(s), vitamin(s) and mineral(s)).

Yet other embodiments may relate to an electronic smoking device 10 comprising an atomizer/liquid reservoir portion for forming a cylindrical tube together with a battery portion 12 of the electronic smoking device. The atomizer/liquid reservoir portion 14 may comprise an air passage and an air inhalation port 36 may be provided at the end of the atomizer/liquid reservoir portion 14 remote from the other end for attachment to the battery portion 12, wherein the atomizer/liquid reservoir portion 14 comprises a receiving portion for receiving a powder for release into the air passage 32 between the atomizer 26 and the air inhalation port 36.

The electronic smoking device 14 may comprise a cartridge 60 comprising a powder reservoir 40 filled with a powder comprising at least one olfactory, gustatory, stimulating and/or nutritive active ingredient (e.g.one or more of: flavor(s), tastant(s), caffeine, carbohydrate(s), vitamin(s) and mineral(s)).

Or, the electronic smoking device 10 may comprise a cartomizer 50 comprising a liquid reservoir 34 for storing a liquid and an atomizer 26 for releasing an aerosol of the liquid from the liquid reservoir 34 into an air passage wherein the cartomizer further comprises a powder reservoir 40 filled with a powder comprising, e.g., flavor(s) and/or tastant(s), caffeine, carbohydrate(s), vitamin(s) and/or mineral(s)).

The embodiments all provide means for delivering at least one olfactory, gustatory, stimulating and/or nutritive active ingredient in a novel way with additional sensory benefits from the effervescent reaction that takes place when the particles are dissolved in the mucous lining of the oral cavity. Another advantage of the invention is that the separation of olfactory, gustatory, stimulating and/or nutritive active ingredient(s) from the e liquid will protect the heating wire from degradation. Thereby a long life-span of the heating coil is maintained. Exemplary olfactory active ingredients comprise one or more flavors and exemplary gustatory active ingredients comprise one or more tastants.

Caffeine is an exemplary active ingredient stimulating the central nervous system. Carbohydrates, vitamins and minerals are exemplary nutritive active ingredients.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### LIST OF REFERENCE SIGNS

- 10: e-cigarette
- 12: battery portion
- 14: atomizer/liquid reservoir portion
- 16: end cap
- 18: battery
- 20: light emitting diode (LED)
- 22: control electronics
- 24: airflow sensor
- 26: atomizer
- 28: heating coil
- 30: wick
- 32: central passage
- 34: liquid reservoir
- 36: air inhalation port
- 38: air inlet
- 40: powder reservoir
- 42: opening
- 44: narrowing
- 50: cartomizer
- 60: cartridge

## Claims

1. An electronic smoking device (10), comprising:
an air passage (32) extending between an air inlet (38) and an air inhalation port (36);
a liquid reservoir (34) for storing liquid for atomization;
an atomizer (26) operable to atomize liquid stored in the liquid reservoir (34) to generate an aerosol and provide said aerosol into said air passage (32); and
a powder reservoir (40) operable to store powder containing at least one olfactory, gustatory, stimulating and/or nutritive active ingredient and release said powder into said air passage (32).

2. The electronic smoking device (10) of claim 1 wherein a narrowing (44) is provided in the air passage (32) and the powder reservoir (40) is arranged to release powder stored in the powder reservoir (40) into air passage (32) within the narrowing (44).

3. The electronic smoking device (10) of claim 2, wherein the narrowing (44) is provided in the air passage (32) between the atomizer (26) and the air inhalation port (36).

4. The electronic smoking device (10) of any preceding claim wherein the powder stored in the powder reservoir (40) and released into the air passage (32) comprises solid particles of at least one olfactory, gustatory, stimulating and/or nutritive active ingredient mixed and/or coated with an effervescent material.

5. The electronic smoking device (10) of claim 4 wherein the effervescent material is effervescent material responsive to humidity as commonly found in human mouths and/or throats.

6. The electronic smoking device (10) of any preceding claim wherein the powder reservoir (40) is arranged to release powder stored in the powder reservoir (40) into the air passage (32) in response to a user inhaling via said air inhalation port (34).

7. The electronic smoking device (10) of any preceding claim wherein the liquid reservoir (34) and/or the powder reservoir (40) is refillable or replaceable.

8. A cartomizer (50) for use with an electronic smoking device in accordance with any preceding claim, comprising:
the liquid reservoir (34) for storing liquid for atomization;
the atomizer (26) for atomizing liquid released from the liquid reservoir (34); and
the powder reservoir (40) filled with powder comprising at least one olfactory, gustatory, stimulating and/or nutritive active ingredient,
wherein the cartomizer is releaseably attachable to a battery portion (12) of an electronic smoking device (10).

9. A cartridge (60) for use with an electronic smoking device in accordance with any of claims 1-7, the cartridge comprising the powder reservoir (40) filled with the powder comprising at least one olfactory, gustatory, stimulating and/or nutritive active ingredient.

10. The cartridge (60) of claim 9 or the cartomizer (50) of claim 8 wherein the powder reservoir (40) has at least one opening (42) operable when open to release powder into an air passage (32) operable to receive an aerosol generated by an atomizer (26).

11. The cartridge (60) of claim 10 or the cartomizer (50) of claim 10 wherein the at least one opening (42) is sealed with a removable seal.

12. An electronic smoking device (10) according to any of claims 1-7, further comprising:
a tubular housing comprising the an air inlet (38) and the air inhalation port (36); wherein the powder reservoir (40) is arranged in the housing.

13. The electronic smoking device of claim 12 with a section of the air passage (32) extending through the powder reservoir (40).

14. The electronic smoking device of claim 13 with a section of the air passage extending centrally through the powder reservoir and through the liquid reservoir.

15. The electronic smoking device of one of claim 12 - 14 with the powder reservoir (40) having one or more outlets into the air passage, and with the outlets between the atomizer and the air inhalation port.
